# EUROPEAN PATENT APPLICATION

(11) **EP 0 792 652 A1**
(43) Date of publication of application: **03.09.1997**
(21) Application number: 97301349.3
(22) Date of filing: 28.02.1997
(51) Int. Cl.: A61L 9/12, A61L 9/04

(54) **Vapour release device**

(30) Priority: 29.02.1996 GB 9604362
(71) Applicant: S.C. Johnson & Son, Inc., Racine, WI 53403-2236 (US)
(72) Inventor: Wetz, Sean, Knightsbridge, London SW1X 0LZ (GB)
(74) Representative: Jones, Alan John

(57) **Abstract**

A vapour dispenser for releasing vapour into the ambien atmosphere comprises a reservoir (1) adapted to be fille with a liquid medium (2) containing a volatile source of th vapour and a vapour dispensing unit (3) for dispensin vapour into the ambient atmosphere. The reservoir (1) an dispensing unit (3) are so disposed that liquid is gravity fed from the reservoir (1) to the dispensing unit (3). Th dispensing unit (3) has a first surface in contact wit liquid from the reservoir and a second surface (7) from which vapour is dispensed, and includes an absorbent laye (5) which restricts the flow of liquid therethrough. Th dispenser is provided with support means such that th dispensing unit (3) is substantially horizontal in use.

## Description

The present invention relates to a device for the controlled release of vapour.

It is known to release volatile materials into the ambient atmosphere from a liquid medium in a container by providing a wick in the upper end of the container into which volatile liquid can be drawn from the container by capillary action, and from which the volatile liquid can evaporate from the exposed end of the wick. As the level of liquid within the container falls, the liquid must travel a greater distance up the wick before it can evaporate. As a result, it is difficult to obtain a uniform release of volatile material with time.

US-A-4 634 614 discloses a device for releasing volatile material from a reservoir through a permeable polymer film, e.g., "Surlyn" (Trademark) film. The device may be suspended so that the polymer film is vertical. The area of the film in contact with the polymer will decrease as volatile material diffuses through the film, thus decreasing the rate at which volatile material diffuses from the device.

It would be possible to obtain a more uniform release of volatile material by feeding liquid to an evaporating surface, e.g. an absorbent material, using a pump operating at a controlled rate of flow. However, this would be complex and expensive.

There is a need for a device for the controlled release of volatile material from a reservoir of liquid which gives a more uniform rate of release of volatile material but avoids the necessity for moving parts.

According to one aspect of the present invention, a vapour dispenser for releasing vapour into the ambient atmosphere comprises a reservoir adapted to be filled with a liquid medium containing a volatile source of the vapour and a vapour dispensing unit for dispensing vapour into the ambient atmosphere, said reservoir and dispensing unit being so disposed that, in use, liquid is gravity-fed to the dispensing unit, said dispensing unit having a first surface for contact with liquid from the reservoir and a second surface from which vapour is dispensed, said device being provided with support means whereby the dispensing unit is disposed substantially horizontally in use.

In a preferred form of the present invention, the dispensing unit comprises
a) a first layer of liquid-permeable material,
b) a second layer of absorbent material, e.g. solvatable polyacrylate or gelled fumed silica, which restricts flow of liquid therethrough, and
c) a third layer comprising a fabric having an interior surface in contact with b) and an exterior surface exposed to the ambient atmosphere in use, said fabric resisting the passage of liquid from b) to said exterior surface.

According to a further aspect of the present invention, there is provided a vapour dispenser as defined above in which the reservoir is filled with liquid medium containing a volatile source of the vapour to be dispensed.

According to another aspect of the present invention, there is provided a vapour dispensing unit for use with a reservoir containing a liquid medium containing a volatile source of the vapour, said dispensing unit comprising
a) a first layer of liquid-permeable material,
b) a second layer of absorbent material, e.g. solvatable polyacrylate polymer or gelled fumed silica, which restricts flow of liquid therethrough, and
c) a third layer comprising a fabric having an interior surface in contact with b) and an exterior surface exposed to the atmosphere in use, said fabric resisting the passage of liquid from b) to said exterior surface.

The liquid medium may be an alkanol, e.g. ethanol or isopropanol, or an alkanol/water mixture. Alkanols such as ethanol can be used to give more concentrated solutions of volatile materials, such as fragrances. The use of water alone as solvent may make it easier to control the rate of flow through the device, but the solvent in which the volatile material is dissolved may be less volatile so that a build up of solvent on the underside of the dispensing unit may be more difficult to avoid.

The volatile material may be a perfume dissolved or dispersed in the liquid medium. Other volatile materials include fragrances, disinfectants, deodorants, air-purifiers, fumigants, perfumants, essences, air-fresheners, insect-repellents, and pharmacological substances such as decongestants.

In a preferred embodiment of the vapour dispenser, the first layer, which in use is in direct contact with the liquid medium in the reservoir, serves to constrain the second layer so as to hold it in place, and can also be used to reduce the flow of liquid into contact with the second layer.

The first layer is liquid-permeable and may be a nonwoven fibrous material, for example paper or a nonwoven synthetic polymer fabric, e.g., made from polypropylene.

The second layer is an absorbent layer, especially a superabsorbent layer such as a layer of solvatable polyacrylate. Polyacrylate polymers, e.g. sodium polyacrylate, are known which are solvatable with solvents, in particular aqueous solvents, and which swell on contact with the solvent. Thus sodium polyacrylate granules which are used to absorb blood and other body fluids in hospitals may be used. They may be obtained in the form of granules or fibres. An alternative second layer is an absorbent layer of gelled fumed silica.

The third layer comprises a fabric which will allow air to circulate through it to the second layer so that vapour evaporating from the second layer can pass through the third layer into the ambient atmosphere. The fabric is however resistant to the passage of liquid from the second layer to the exterior surface of the fabric third layer.

Such fabrics are used as the body-facing layer, the so-called "stay dry" layer or "top weave", of disposable nappies (babies' diapers) or sanitary towels.

It is preferred for the dispensing unit to extend across at least part of the base of the reservoir. Preferably the dispensing unit extends across substantially all the base of the reservoir.

The support means may be a hook or aperture by which the device may be suspended from an external support. Preferably the device is provided with support means extending below the dispensing unit so that the second surface of the dispensing unit can be supported above a flat surface.

It may be desirable to provide baffle means, with one or more apertures but otherwise impermeable, extending across the base of the reservoir to restrict the flow of liquid into the dispensing unit.

It is also desirable to ensure good sealing between the dispensing unit and the reservoir to reduce the rate at which air can diffuse into the vapour dispenser. This will in turn reduce the rate at which liquid carrying the volatile material can flow out of the reservoir.

The present invention will now be described, by way of example, with reference to the accompanying diagrammatic drawings in which:
Figure 1 is perspective view from below and partly in section of one embodiment of a vapour dispenser according to the invention; and
Figure 2 is an exploded perspective view of another embodiment of vapour dispenser according to the invention.

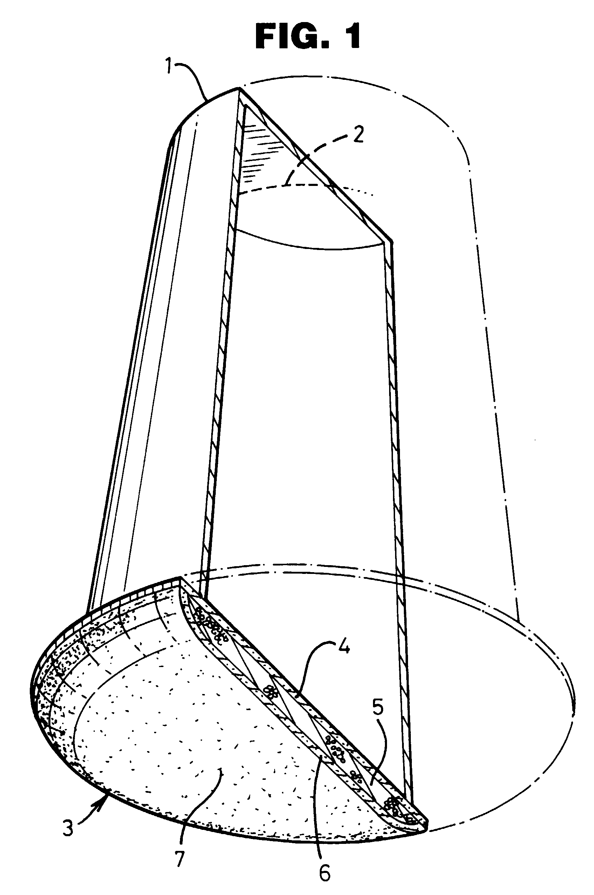

Referring to Figure 1, the vapour dispenser comprises a reservoir (1), e.g. of transparent or translucent plastics material, for liquid (2) comprising a volatile material. At the base of the reservoir is attached, as by bonding such as by adhesive bonding, a dispensing unit (3) as described below. The vapour dispenser may be placed on a stand (not shown) to support it clear of a surface on which it is placed whereby the dispensing unit is disposed substantially horizontally in use. In use, the liquid (2) passes under the influence of gravity into the dispensing unit (3). One liquid (2) which may be used in the embodiment shown is a solution or dispersion of the volatile material in ethanol.

The dispensing unit (3) is a layered structure comprising a first layer (4) of liquid-permeable material, namely a pad formed of a nonwoven polypropylene fabric. This acts to restrict liquid flow into the dispensing unit, and to retain a layer (5) of superabsorbent material such as sodium polyacrylate granules as commercially available for absorbing liquid spillages. Alternatively gelled fumed silica may, for example, be used as absorbent layer (5).

The layer of material (5) lies over a layer (6) of material corresponding to the body-facing layer, the so-called "top weave" or "stay dry" layer, used in babies' nappies (diapers), and was obtained by taking apart a commercially available disposable nappy (diaper). The interior surface (not seen in Figure 1) of the fabric layer (6) is in contact with layer (5), and the lower, exterior surface (7) of fabric layer (6) is exposed to the ambient atmosphere.

Vapour is dispensed from surface (7) into the ambient atmosphere. The fabric layer (6) resists the passage of liquid (2) to the exterior surface (7).

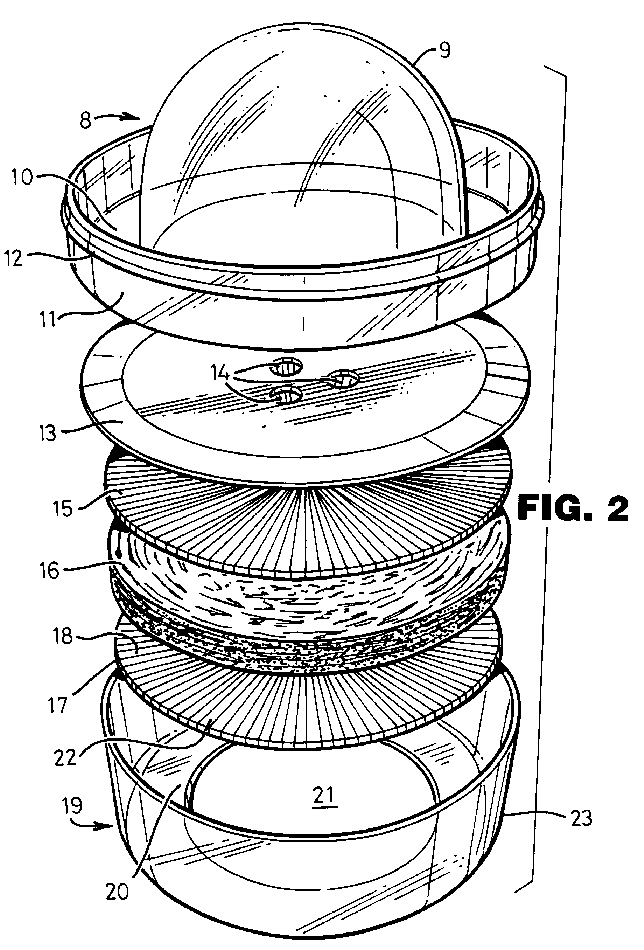

Referring now to Figure 2, an alternative vapour dispenser comprises a hat-shaped upper member (8) moulded from plastics material, preferably transparent or translucent plastics material, the central dome (9) of which constitutes the reservoir of the dispenser. The amount of liquid remaining in the reservoir at any one time can thus be determined simply by visual inspection.

Hat-shaped member (8) includes a "brim" (10) including an upstanding peripheral wall (11) provided with an inverted u-shaped peripheral socket (12), the purpose of which will be described hereinbelow.

In this embodiment, the dispensing unit is also a layered structure comprising a baffle plate (13) having three apertures (14), a first layer (15) of "stay-dry" fabric, a second layer constituted by a pad (16) of superabsorbent sodium polyacrylate fibre, and a third layer (17) of "stay-dry" fabric, the upper, interior surface (18) of which is in contact with layer (16).

A cup-shaped sealing member (19), e.g. of plastics material, surrounds the dispensing unit and seals it to the reservoir (9). Sealing member (19) has an inwardly-extending flange (20) which defines a central aperture (21) in member (19). The aperture (21) leaves exposed to the ambient atmosphere most of the lower, exterior surface (22) of layer (17), and provides the surface from which vapour is dispensed. Sealing member (19) also comprises an upwardly-extending rim (23) which surrounds the dispensing unit and forms a snap-fit or push-fit ("friction-fit") in socket (12) of member (8), thus maintaining the overall unitary integrity of the vapour dispenser.

## Claims

1. A vapour dispenser for releasing vapour into the ambient atmosphere which comprises a reservoir adapted to be filled with a liquid medium containing a volatile source of the vapour and a vapour dispensing unit for dispensing vapour into the ambient atmosphere, said reservoir and dispensing unit being so disposed that, in use, liquid is gravity-fed to the dispensing unit, said dispensing unit having a first surface for contact with liquid from the reservoir and a second surface from which vapour is dispensed, said dispenser being provided with support means whereby the dispensing unit is disposed substantially horizontally in use.

2. A vapour dispenser according to claim 1 wherein the reservoir contains a liquid comprising a volatile material.

3. A vapour dispenser according to claim 1 or claim 2 wherein the dispensing unit comprises
a) a first layer of liquid-permeable material,
b) a second layer of absorbent material which restricts flow of liquid therethrough, and
c) a third layer comprising a fabric having an interior surface in contact with b) and an exterior surface exposed to the ambient atmosphere in use, said fabric resisting the passage of liquid from b) to said exterior surface.

4. A vapour dispenser according to claim 1 or claim 2 wherein the dispensing unit comprises
a) a first layer of permeable material which allows liquid to pass through it,
b) a second layer of solvatable polyacrylate which restricts flow of liquid through it, and
c) a third layer comprising a fabric having an interior surface in contact with the polyacrylate and an exterior surface exposed to the atmosphere in use, said fabric resisting the passage of liquid from the polyacrylate to said exterior surface.

5. A vapour dispenser according to any one of claims 1 to 4 which is provided with support means extending below the dispensing unit whereby the second surface of the dispensing unit can be supported above a flat surface.

6. A vapour dispenser according to any one of claims 1 to 5 further including baffle means with at least one aperture, said baffle means extending across the base of the reservoir to restrict the flow of liquid into the dispensing unit.

7. A vapour dispensing unit for use with a reservoir containing a liquid medium containing a volatile source of the vapour, said dispensing unit comprising
a) a first layer of liquid-permeable material,
b) a second layer of absorbent material which restricts flow of liquid therethrough, and
c) a third layer comprising a fabric having an interior surface in contact with b) and an exterior surface exposed to the ambient atmosphere in use, said fabric resisting the passage of liquid from b) to said exterior surface.

8. A vapour dispensing unit for use with a reservoir containing a liquid medium containing a volatile source of the vapour, said dispensing unit comprising
a) a first layer of permeable material which allows liquid to pass through it,
b) a second layer of solvatable polyacrylate which restricts flow of liquid through it, and
c) a third layer comprising a fabric having an interior surface in contact with the polyacrylate and an exterior surface exposed to the atmosphere in use, said fabric resisting the passage of liquid from the polyacrylate to said exterior surface.
